Europäisches Patentamt

(19) **European Patent Office** (11) Publication number: **0 010 443**

Office européen des brevets **B1**

(12) ## EUROPEAN PATENT SPECIFICATION

(45) Date of publication of patent specification: **20.10.82**

(51) Int. Cl.³: **C 07 C 139/00, C 07 C 143/55**

(21) Application number: **79302281.5**

(22) Date of filing: **19.10.79**

(54) **Process for preparing sodium-m-nitrobenzenesulfonate.**

(30) Priority: **24.10.78 US 954316**

(43) Date of publication of application:
**30.04.80 Bulletin 80/9**

(45) Publication of the grant of the patent:
**20.10.82 Bulletin 82/42**

(84) Designated Contracting States:
**CH DE FR GB IT**

(56) References cited:
**CH - A - 478 772
DE - A - 2 328 574
GB - A - 1 030 308
US - A - 3 492 342**

(73) Proprietor: **E.I. DU PONT DE NEMOURS AND COMPANY**
**Legal Department 1007 Market Street**
**Wilmington Delaware 19898 (US)**

(72) Inventor: **Baum, Arthur Aloysius**
**2311 Newport Gap Pike**
**Wilmington, Delaware 19808 (US)**
Inventor: **Rothman, Leonard Aaron**
**3 E. Mill Drive**
**Great Neck, New York 11021 (US)**

(74) Representative: **Woodcraft, David Charles et al,**
**BROOKES & MARTIN High Holborn House 52/54**
**High Holborn**
**London, WC1V 6SE (GB)**

Courier Press, Leamington Spa, England.

## Process for preparing sodium m-nitrobenzenesulfonate

This invention relates to an improved process for preparing sodium m-nitrobenzenesulfonate by sulfonation of nitrobenzene using oleum.

As was noted in British Specification 1,030,308, it has been conventional to use a large excess of sulfuric acid or oleum as a sulfonating agent and a solvent in the sulfonation of nitro-aromatic compounds, resulting in the product being obtained in admixture with a large amount of sulfuric acid. The nitro-aromaticsulfonic acid was isolated from the reaction mixture by reacting the same with lime so as to convert the excess sulfuric acid to calcium sulfate which was removed by filtration. In the alternative, the nitro-aromaticsulfonic acid was isolated by the sodium chloride "salting out" method. As pointed out in the British Specification, the former method is an uneconomic one; large amounts of calcium sulfate are formed which must be washed with large amounts of water, and then the filtrate must be evaporated so as to recover the product. The "salting out" method has its disadvantages as well, as are further described in the British patent.

A process for sulfonating nitro-aromatic compounds, including nitrobenzene, with $SO_3$, so as to produce the sodium salt is disclosed in U.S. Patent 3,492,342. While the use of $SO_3$ as a sulfonating agent avoids the production of large amounts of sulfuric acid, thereby dealing with a recovery problem common to this art, it has disadvantages as well. Such sulfonations with $SO_3$ are attended by sulfone formation, resulting in the consumption of significant quantities of reactants. Moreover, the $SO_3$ has a tendency to polymerize. It is therefore necessary to supply heat to the equipment being used to dispense the $SO_3$, so as to prevent plugging of the equipment by the polymers which are formed. In addition, the presence of a reasonable amount of sulfuric acid can be an advantage in processes for preparing sulfonates such as sodium m-nitrobenzenesulfonate, because it is soluble therein.

Fiat Final Report No. 1313, dated February 1, 1948, PB 85172, pages 187—191, discloses a method for preparing metanilic acid, starting with nitrobenzene, 98% sulfuric acid and 65% oleum (equivalent to 44% oleum). After the sulfonation reaction was completed, the reaction mass was diluted with water, and sodium sulfate and calcium carbonate ("carbid kalk") were added. Assuming that substantially all of the nitrobenzene was converted to the sulfonic acid, the amount of sodium sulfate provided was insufficient to convert all of it to sodium m-nitrobenzenesulfonate.

According to the present invention there is provided a process for the preparation of sodium m-nitrobenzenesulfonate which comprises reacting slightly more than one mole of $SO_3$, as 65% oleum, per mole of nitrobenzene at a temperature between 80° and 130°C for a period of time sufficient to effect sulfonation of substantially all of the nitrobenzene; thereafter, at a temperature not higher than 105°C., combining the sulfonation reaction mass with sufficient caustic soda to convert substantially all of the sulfuric acid and the resulting m-nitrobenzenesulfonic acid to $Na_2SO_4$ and sodium m-nitrobenzenesulfonate; at a pH between 7 and 8.6 and at a temperature between 80°C and 105°C, sufficient water is provided by, or added to, the resulting mixture of salts that at least 60% of the $Na_2SO_4$ is insoluble therein and the sodium m-nitrobenzenesulfonate is dissolved therein; and separating the insoluble $Na_2SO_4$ from the reaction mass at 80° to 105°C.

In the sulfonation reaction of this invention, one uses a slight excess, e.g., 5 mole % $SO_3$, as 65% oleum, over the theoretical amount required. With careful handling, particularly efficient mixing, it is possible to combine the nitrobenzene with the oleum, starting at room temperature, relying on the exothermic nature of the reaction to provide the heat needed to effect substantially complete reaction of the nitrobenzene and the oleum. Nevertheless, it is advisable, from the standpoint of safety, to preheat the nitrobenzene to a temperature between 80° and 130°C and then combine it with the 65% oleum. In any event, it is necessary that the temperature in the sulfonator be at least 80°C. Otherwise, the sulfonation mass could solidify. On the other hand, the sulfonation mass will decompose violently at 200°C or above. Consequently, one should operate the process at a temperature not exceeding 130°C. Preferably, one would preheat the nitrobenzene to a temperature between 100° and 110°C and combine the oleum and the nitrobenzene while holding the temperature in that range, supplying cooling as needed.

After all of the oleum and nitrobenzene have been combined with one another, the sulfonation mass is held at a temperature between 80° and 130°C, preferably 100°—110°C for a period of time sufficient to assure substantial completion of the reaction. The period of time needed for completion of sulfonation will, of course, depend upon the amount of nitrobenzene and the temperature at which the sulfonation reaction is conducted. Thus, the reaction goes more rapidly at higher temperature. While the initial mixing of the oleum and nitrobenzene is exothermic, the reaction thereafter requires that heat be supplied intermittently. Every effort should be made to convert substantially all of the nitrobenzene to the m-nitrobenzenesulfonic acid during the sulfonation reaction, since it would be difficult to remove the nitrobenzene later in the process.

Following completion of the sulfonation reaction the reaction mass should be combined with sufficient caustic soda to convert substantially all of the m-nitrobenzenesulfonic acid and substantially all of the sulfuric acid in the system to sodium m-nitrobenzenesulfonate and $Na_2SO_4$. In practice, a slight excess of caustic soda is used so as to assure substantially complete conversion of all of those acids to those salts. The amount of excess caustic soda is kept to a minimum, as it is necessary to neutralize substantially all of it before the final product is recovered.

The sulfonation mass should be transferred to the aqueous caustic soda at a temperature between 80° and 130°C, preferably 85° to 110°C. Cooling should be supplied to the vessel in which the sulfonation mass and the aqueous caustic soda are combined, since they combine exothermically. Usually, the components are kept at a temperature below 105°C, as the charge may foam, preferably 95° to 100°C maximum. Prior to combining the sulfonation mass with it, the aqueous caustic soda may be at any temperature above its freezing point and below 105°C, preferably about 20°C. The combined sulfonation mass and aqueous caustic soda should be maintained in the vessel in which they are combined for a period sufficiently long so that substantially all acidity has been eliminated. Moreover, maintaining that charge at those temperatures will rid the charge of the last traces of nitrobenzene. Thereafter, substantially all excess alkalinity, if any, should be reduced to a pH of 7—8.6, for example, by adding sulfuric acid to the system.

At a pH between 7 and 8.6, and at a temperature between 80° and 105°C, sufficient water is provided by, or added to, the resulting mixture of salts, so that at least 60% of the $Na_2SO_4$ is insoluble therein, while substantially all of the sodium m-nitrobenzenesulfonate remains dissolved therein. The difference in the solubilities of the two salts forms a basis for their separation. One can remove the $Na_2SO_4$ by conventional means, e.g. filtering, centrifuging, decanting, and the like. Preferably a filter press, preheated with steam in which the charge is filtered at a temperature between 95° and 100°C, is used.

This invention not only results in lower raw material costs, but it makes possible labor and other material savings because larger amounts of the final product can be manufactured in existing equipment in which one heretofore used a large excess of sulfuric acid as a solvent. Yields of the desired product are higher, and smaller amounts of by-products are formed the latter contributing to better pollution control. As smaller amounts of sulfuric acid are involved in the process and as only one filtration step is required, large excesses of water need not be evaporated.

This invention is further illustrated in the example that follows, in which, unless otherwise specified percentages and parts are by weight and temperature is in degrees Celsius.

**Example**

Into a cast iron jacketed sulfonator, equipped with an agitator, were charged 2600 parts (21.12 moles) of nitrobenzene which was heated to a temperature in the range of between 100 and 110°C. Over a period of 2 hours, 2730 parts (22.16 moles) of 65% oleum were added with agitation to the nitrobenzene while maintaining the temperature in the range between 100° and 110°C by supplying cooling liquid to the jacket of the sulfonator. Mixing of the oleum and nitrobenzene was continued for 8 hours, intermittently supplying heated fluid to the jacket so as to maintain the temperature between 100° and 110°C. To the drowning tub (a cast iron kettle equipped with carbon steel coils which can be used to heat with steam or cool with water) were added 1238 parts of water and 5738 parts of a 30% aqueous solution of caustic soda (1721 parts or 43.03 moles of NaOH). The sulfonation mass was added to the drowning tub. The temperature of the contents of the drowning tub rose to 95°C, whereupon cooling water was passed through the coils to maintain the temperature at a maximum of 95°C during the 3-hour period used for completion of the drowning. The drowned mass did not smell of nitrobenzene. The pH was adjusted to 7.8±0.8 with 91 parts of 97% sulfuric acid. The contents of the drowning tub were transferred to a filter press which had been preheated with steam, and the charge to the filter press was filtered at 95°—100°C over a period of 2 hours. It was found that filtration proceeded at a faster rate when no agitation was supplied to the drowning tub. The filtrate weighed 10,005 parts, and upon analysis by titanous titration, 43.9% was determined to be sodium m-nitrobenzenesulfonate; it also contained 4.4% $Na_2SO_4$ and 0.2% "sulfone". The filter cake contained 1044 parts $Na_2SO_4$.

**Claims**

1. A process for the preparation of sodium m-nitrobenzenesulfonate which comprises reacting slightly more than one mole of $SO_3$, as 65% oleum, per mole of nitrobenzene at a temperature between 80°C and 130°C for a period of time sufficient to effect sulfonation of substantially all of the nitrobenzene; thereafter at a temperature not higher than 105°C., combining the sulfonation reaction mass with sufficient caustic soda to convert substantially all of the sulfuric acid and the resulting m-nitrobenzenesulfonic acid to $Na_2SO_4$ and sodium m-nitrobenzenesulfonate; at a pH between 7 and 8.6 and at a temperature between 80° and 105°C, sufficient water is provided by, or added to, the resulting mixture of salts that at least 60% of the Na$_2$SO$_4$ is insoluble therein and the sodium m-nitrobenzenesulfonate is dissolved therein; and separating the insoluble Na$_2$SO$_4$ from the reaction mass at 80° to 105°C.

2. A process according to Claim 1 wherein the nitrobenzene and the oleum are combined at a temperature between 100°C and 110°C and the sulfonation reaction is performed at said temperature.

3. A process according to Claim 1 or Claim 2 wherein the sulfonation mass is combined with aqueous caustic soda having a temperature of about 20°C, and the heat of the reaction is permitted to bring the reaction temperature to a maximum temperature between 95°C and 100°C.

4. A process according to any one of the preceding claims wherein the Na$_2$SO$_4$ is separated from the reaction mass in the solid state at a temperature between 95°C and 100°C.

5. A process according to any one of the preceding claims wherein the SO$_3$ is used in an excess of about 5 molar% over the theoretical amount required for complete sulfonation of the nitrobenzene.

**Revendications**

1. Procédé de préparation de m-nitro-benzènesulfonate de sodium qui consiste à faire réagir un peu plus d'une mole de SO$_3$, sous forme d'oléum à 65%, par mole de nitrobenzène, à une température comprise entre 80 et 130°C, pendant un laps de temps suffisant pour effectuer la sulfonation de substantiellement tout le nitrobenzène; ensuite, à une température ne dépassant pas 105°C, à combiner la masse réactionnelle de sulfonation à suffisamment de soude caustique pour convertir pratiquement tout l'acide sulfurique et l'acide m-nitrobenzènesulfonique obtenu en Na$_2$SO$_4$ et m-nitrobenzènesulfonate de sodium; à un pH compris entre 7 et 8, 6 et à une température comprise entre 80 et 105°C, le mélange de sels obtenu fournit, ou on y ajoute suffisamment d'eau pour qu'au moins 60% du Na$_2$SO$_4$ soit insoluble dans celle-ci et que le m-nitroben-zènesulfonate de sodium s'y dissolve; et à séparer le Na$_2$SO$_4$ insoluble de la masse réactionnelle entre 80 et 105°C.

2. Procédé selon la revendication 1, dans lequel on combine le nitrobenzène et l'oléum à une température comprise entre 100 et 110°C et on effectue la réaction de sulfonation à cette température.

3. Procédé selon la revendication 1 ou 2, dans lequel on combine la masse de sulfonation à de la soude caustique aqueuse ayant une température d'environ 20°C et on laisse la chaleur de la réaction porter la température de réaction à une température maximale comprise entre 95 et 100°C.

4. Procédé selon l'une quelconque des revendications précédentes, dans lequel on sépare le Na$_2$SO$_4$ de la masse réactionnelle à l'état solide à une température comprise entre 95 et 100°C.

5. Procédé selon l'une des revendications précédentes, dans lequel on utilise le SO$_3$ en un excès d'environ 5 moles% par rapport à la quantité théorique nécessaire à la sulfonation complète du nitrobenzène.

**Patentansprüche**

1. Verfahren zur Herstellung von Natrium-m-nitrobenzolsulfonat durch Reaktion von ein wenig mehr als 1 Mol SO$_3$, als 65% Oleum, pro Mol Nitrobenzol bei einer Temperatur zwischen 80°C und 130°C während eines ausreichenden Zeitraums zur Bewirkung der Sulfonierun von im wesentlichen dem gesamten Nitroben-zol; anschliessende Kombination der Sulfonier-ungsreaktionsmasse bei einer Temperatur von nicht höher als 105°C mit ausreichend kaustischer Soda zur Umwandlung von im wesentlichen der gesamten Schwefelsäure und der resultierenden m-Nitrobenzolsulfonsäure in Na$_2$SO$_4$ und Natrium-m-nitrobenzolsulfonat; Bereitstellen oder Zusatz von ausreichend Wasser durch das resultierende bzw. zu dem resultierenden Salzgemisch bei einem pH-Wert zwischen 7 und 8, 6 und bei einer Temperatur zwischen 80° und 105°C, so dass mindestens 60% des Na$_2$SO$_4$ darin unlöslich sind und das Natrium-m-nitrobenzolsulfonat darin gelöst wird; und Abtrennen des unlöslichen Na$_2$SO$_4$ aus der Reaktionsmasse bei 80° bis 105°C.

2. Verfahren nach Anspruch 1, bei dem das Nitrobenzol und das Oleum bei einer Temperatur zwischen 100°C und 110°C kombiniert werden und die Sulfonierungsreaktion bei dieser Temperatur durchgeführt wird.

3. Verfahren nach Anspruch 1 oder 2, bei dem die Sulfonierungsmasse mit wässriger kaustischer Soda, die eine Temperatur von etwa 20°C aufweist, kombiniert wird und die Reaktionswärme die Reaktionstemperatur auf eine maximale Temperatur zwischen 95°C und 100°C bringen kann.

4. Verfahren nach einem der vorhergehenden Ansprüche, bei dem das Na$_2$SO$_4$ aus der Reaktionsmasse im festen Zustand bei einer Temperatur zwischen 95°C und 100°C abgetrennt wird.

5. Verfahren nach einem der vorhergehenden Ansprüche, bei dem das SO$_3$ in einem Über-schuss von etwa 5 Mol-% über der theoretischen Menge verwendet wird, die zur vollständigen Sulfonierung des Nitrobenzols erforderlich ist.